# EUROPEAN PATENT APPLICATION

(11) **EP 4 261 230 A1**
(43) Date of publication of application: **18.10.2023**
(21) Application number: 20958484.6
(22) Date of filing: 13.11.2020
(51) Int. Cl.: C07K 19/00, C12N 15/62, C12N 15/81, C12N 1/19, A61K 47/64, A61K 45/00, A61P 35/00

(54) **RECOMBINANT HUMAN SERUM ALBUMIN-COLLAGEN BINDING DOMAIN FUSION PROTEIN FOR TUMOR-SPECIFIC TARGETING MATRIX AND APPLICATION THEREOF**

(30) Priority: 21.10.2020 CN 202011129275
(71) Applicant: Fortunerock (China) Co., Limited All Rights Reserved., Beijing 100026 (CN); Tianjin Sinobiotech Ltd., Tianjin 300457 (CN); BEIJING BIO-FORTUNE TECHNOLOGY LIMITED, Beijing 100026 (CN); TIANJIN LINDA SINOBIOTECH CO., LTD, Tianjin 300457 (CN)
(72) Inventor: YU, Zailin, Beijing 100026 (CN); FU, Yan, Beijing 100026 (CN); YANG, Xiaonan, Beijing 100026 (CN); FU, Yusong, Beijing 100026 (CN); QIAO, Guoqiang, Beijing 100026 (CN); YANG, Bo, Beijing 100026 (CN); CHEN, Ying, Beijing 100026 (CN); HOU, Qiong, Beijing 100026 (CN)
(74) Representative: Isern Patentes y Marcas S.L.
(86) International application number: PCT/CN2020/128652
(87) International publication number: WO 2022/082906

(57) **Abstract**

Provided is a recombinant human serum albumin-collagen binding domain (CBD) fusion protein for tumor-specific targeting matrix and its use in specific targeted treatment of a solid tumor. HSA and the CBD generate gene fusion in the form of different molecular structures, and can be expressed in a constructed recombinant engineered host. A pharmaceutical recombinant fusion protein is obtained through large-scale fermentation. The recombinant fusion protein shows tumor-specific targeting of solid tumors and can be used as a starting material of a broad-spectrum matrix carrier. The recombinant fusion protein is coupled with any tumor treatment drug, small-molecule compound, or cytotoxic protein to form an innovative pharmaceutical composition. The pharmaceutical composition of the recombinant fusion protein is also long-acting, can prolong a half-life of the conjugate drug *in vivo,* and has a significantly better clinical curative efficacy on anti-tumor than that of a monomer drug.

## Description

### CROSS-REFERENCE TO RELATED APPLICATION

The present application claims priority to the Chinese Patent Application No. 2020111292757, filed with the China National Intellectual Property Administration (CNIPA) on October 21, 2020, and entitled "RECOMBINANT HUMAN SERUM ALBUMIN-COLLAGEN BINDING DOMAIN FUSION PROTEIN FOR TUMOR-SPECIFIC TARGETING MATRIX, AND USE THEREOF", which is incorporated herein by reference in its entirety.

### TECHNICAL FIELD

The present disclosure belongs to the technical field of biomedicine, and specifically relates to a recombinant human serum albumin (HSA)-collagen binding domain (CBD) fusion protein for tumor-specific targeting matrix, and use thereof. HSA and the CBD generate gene fusion in the form of different molecular structures, and can be expressed in constructed recombinant engineered host. The pharmaceutical grade recombinant fusion protein is produced through large-scale fermentation. The recombinant fusion protein shows tumor-specific targeting of solid tumors and can be used as a starting material of a broad-spectrum matrix carrier. The recombinant fusion protein is coupled with any tumor treatment drug, small-molecule compound, or cytotoxic protein to form an innovative pharmaceutical composition. In the present disclosure, the pharmaceutical composition of the recombinant fusion protein is also long-acting, can prolong a half-life of the conjugate drug *in vivo,* and has a significantly better clinical curative efficacy on anti-tumor than that of a monomer drug. The present disclosure further provides use of the pharmaceutical composition in preparation of a drug for specific targeted treatment of a solid tumor, and a preparation method of a corresponding drug.

### BACKGROUND

Human serum albumin (HSA) is a soluble monomeric protein that constitutes half of the total protein in blood. Albumin is the main component of blood, with a content of 40 grams per liter of blood in the human body, and the half-life of albumin is 14 days to 20 days. As a basic carrier, albumin serves as an essential carrier for the delivery of fatty acid, steroid, and hormone molecules. Stable inert nature of the albumin is an important factor in the maintenance of blood pressure. Serum albumin is a globular, nonglycosylated serum protein with a molecular weight of 65 kD and having 585 amino acids. This protein (albumin precursor) is then processed by Golgi apparatus to remove a guiding peptide and secreted out of the cells. Serum albumin has 35 cysteines. In blood, albumin is a monomer with 17 disulfide bonds. When no polypeptides are secreted, albumin products in yeast cells are in a mismatched state. At this point, the albumin products may lose 90% of their antigenicity (compared to albumin of a natural state in plasma), and then form insoluble albumin aggregates, which do not show a biological activity of albumin. The albumin used clinically is extracted from human plasma. A method of recombinantly expressing human serum albumin (rHSA) using microorganisms has been disclosed in Chinese authorized patent ZL200410057313.7 (YU Zailin and FU yan).

In the composition of pharmaceutical preparations, albumin is also generally used as a stabilizer for drugs, especially in the manufacture of biological drugs and vaccines. In summary, HSA and a therapeutic protein are genetically fused by genetic engineering technology, and then expressed as a recombinant fusion protein in yeast or vertebrate cells. This recombinant fusion protein can show a great advantage in resisting enzymatic hydrolysis *in vivo* and can greatly improve a lifespan of the therapeutic protein *in vivo* and *in vitro.* That is to say, this recombinant fusion protein can increase stability and a longer half-life of the therapeutic protein in serum and during storage, so as to achieve long-acting protein drugs. In this way, the frequency of administration can be greatly reduced, and a better therapeutic effect can be obtained in the clinical treatment of major diseases.

HSA also has the function of non-specific accumulation on solid tumors. Since the solid tumors proliferate rapidly, their tumor body can generate a large number of microvessels to provide a large blood supply, and albumin can lead to accumulation in local microvessels. With the help of this specific function of albumin, scientists have developed paclitaxel for injection (albumin-bound type). This drug has a trade name: Abraxane^{®} innovative drug (American Pharmaceutical Partners, Inc., USA), and is prepared by coupling of the paclitaxel with albumin. After coupling paclitaxel with albumin, the toxic and side effects of paclitaxel can be greatly reduced, and a remarkable clinical efficacy is obtained.

Collagen-binding domain (CBD) is a part of the von Willebrand Factor (vWF) of human von Willebrand disease type A. vWF mediates the adhesion of platelets to vascular fibril collagen, and plays an extremely important role in the process of hemostasis. A mature vWF protein contains 2,050 amino acids and is also a key protein that binds to fibrillar collagen. This protein has multiple domains, among which the A3 domain mediates platelet aggregation.

vWF-A3 domain (also known as CBD) refers to a peptide segment between 920 to 1111 of a mature peptide, with a molecular weight of 20 kD. The articles "Huizinga E G, Martijn V D P R, Kroon J, et al. Crystal structure of the A3 domain of human von Willebrand factor: implications for collagen binding.[J]. Structure, 1997, 5(9):1147." and "Brondijk T H C, Bihan D, Farndale R W, et al. Implications for collagen I chain registry from the structure of the collagen von Willebrand factor A3 domain complex.[J]. Proceedings of the National Academy of Sciences of the United States of America, 2012, 109(14):5253-8." describe in detail a crystal structure formed by binding the vWF A3 domain to a type III homotrimeric polypeptide collagen, and further infer a binding site of the vWF A3 domain to a type I heterotrimer. The type III homotrimeric polypeptide collagen partially overlaps with the binding site of vWF A3 domain and osteonectin and discodin domain receptor 2.

Solid tumors proliferate rapidly, and their tumor body can generate a large number of microvessels, requiring a large amount of blood supply, such that albumin can accumulate in the local microvessels of the tumor body. The inner walls of these microvessels have specially proliferated fibrous collagen bodies, which also lead to greatly enhanced opportunities for vascular collagen to be tightly combined with the CBD of vWF. Sasaki et al. in August 2019 [Sasaki et al., Sci. Adv. 2019; 5:1-12] published the fusion of mouse serum albumin (MSA) with CBD. The CBD is fused to an N-terminal of the MSA through a connecting peptide, and a His-tag (6-His) is added to a C-terminal of the MSA of a resulting fusion protein. This fusion protein is then expressed in vertebrate cells (HEK293F). However, the serum albumin in this fusion protein is not of human origin, and a connecting peptide is provided between the C-terminal of CBD and the N-terminal of mouse albumin. Moreover, the fusion protein also contains a 6His-tag. Meanwhile, HEK293 cells are used to prepare the fusion protein disclosed in the literature. This expression cell system is a non-industrialized cell system, with a yield per liter being only in the microgram to milligram range. The recombinant fusion protein obtained in this way is difficult to meet the production requirements of pharmaceutical grade/kg, and shows a considerable disadvantage in production cost.

Chemotherapy drugs, as small-molecule drugs and toxic proteins, are key clinical drugs for inhibiting tumor cell growth and tumor body shrinkage. Clinically familiar small-molecule chemotherapy drugs mainly include a platinum drug, a nitrosourea drug, an antifolate drug, an antipyrimidine drug, a vinblastine drug, a taxane drug, a camptothecin drug, an anthracycline drug, a drug that disrupts a structure and a function of a DNA, a drug that is intercalated into a DNA and interferes with transcription of the DNA, and a drug that affects protein synthesis. Clinically, tumor cytotoxic proteins preferably include: ricin, diphtheria toxin, trichosanthin (Apotin) also known as VP3 protein, *Pseudomonas aeruginosa* exotoxin, inhibitor of apoptosis protein (Survivin), human epidermal growth factor (EGF), gonadotrophin-releasing hormone (GnRH), and deoxyribonuclease II (Dnase II). These proteins are not drug-targeting, show extremely high dosages required in clinical practices, and have highly significant systemic toxic and side effects caused by non-targeting. If the above small-molecule drugs are carried by solid tumor-specific targeting matrix carriers, the dosage of toxic small-molecule drugs in clinical practice can be greatly reduced, and the toxic and side effects of systemic chemotherapy drugs can also be significantly and greatly reduced. Accordingly, the research and development of new tumor treatment-specific targeting drugs is the most concerned research direction of tumor treatment.

A method for chemical coupling of protein and small-molecule compound to prepare pharmaceutical composition can be used for coupling the recombinant fusion protein and small-molecule compound involved in the present disclosure, mainly including direct coupling. The albumin-paclitaxel binding (coupling) is taken as an example: an albumin stock solution is allowed to stand at room temperature for 1 h, 4 ml of the albumin stock solution is added into a centrifuge tube, and a paclitaxel stock solution of N1 :N2=9:1 (where N1 is a molar weight of the paclitaxel, and N2 is a molar weight of the albumin) into the centrifuge tube. A resulting mixture is allowed to stand overnight, and then centrifuged at 3,000 rpm for 20 min. A resulting supernatant is filtered through a 0.22 µm membrane filter. There is also a paclitaxel-albumin complex in the supernatant. If nanoparticles of the complex are prepared, a method reported by Gong Guangming et al. (Nanotechnology, 22: 295-603, 2011; Biotechnology World, 2015, article number: 1674-2060 (2015) 08-0164-01) can be adopted to directly prepare nanoparticles of an albumin drug compound, and the nanoparticles are coupled with a small-molecule compound. For example, 100 mg of albumin is dissolved in 10 ml of water, 3-mercapto-2-butanone (at a final concentration of 10 µM) is added in a water bath at 37°C, 30 mg of paclitaxel is added after 10 min, and an obtained mixture is stirred for another 10 min. An obtained reaction solution is dialyzed through a 0.22 µM membrane to remove uncoupled paclitaxel, such that a nanoparticle dosage form of the albumin/small-molecule compound drug can be obtained.

The recombinant fusion protein-coupled complex in the tumor site of an animal body can be generally detected by a conventional protein labeling method, so as to trace and detect the distribution of a specific targeting treatment drug after coupling to solid tumors *in vivo.* For example:
1) Radioactive ¹²⁵I labeling of the recombinant fusion protein-coupled complex: a qualified unit is entrusted to conduct ¹²⁵I labeling using an ammonium chloride T labeling method. A labeled test product is purified, its purity is verified by SEEC-HPLC, and determination is conducted on distribution of the recombinant fusion protein-coupled complex in the animal body.
2) PET-CD method: an ⁸⁹Zr radiolabeled protein can be directly used in the radioactive tracer labeling of recombinant fusion protein-coupled complex formed by the recombinant fusion protein and the tumor treatment drug (such as small-molecule drugs or toxic proteins). Deferoxamine (DFO) is the most common ⁸⁹Zr⁴⁺ labeling chelator. DFO is a hexaligand iron chelating agent, which can form stable complexes with metals. A [⁸⁹Zr] zirconium oxalate solution is adjusted to a pH value of 7 with Na₂CO₃ or HEPES buffer, a CBD-HSA recombinant fusion protein-coupled complex containing DFO is added and allowed reacted at room temperature for 30 min to 60 min. Finally, unlabeled zirconium oxalate [⁸⁹Zr] is separated and removed by molecular exclusion packing or SEC-HPLC to obtain a purified product. This method can afford a labeling yield of 35% to 98%.

These known techniques, as practical supporting references of the present disclosure, can be used to prepare pharmaceutical compositions obtained in the present disclosure by coupling the recombinant fusion protein with small-molecule compounds, and further prepare these pharmaceutical compositions into pharmaceutical dosage forms that can be used for clinical treatment.

### SUMMARY

In view of this, an objective of the present disclosure is to provide a recombinant human serum albumin (HSA)-collagen binding domain (CBD) fusion protein for a tumor-specific targeting matrix carrier, and use thereof. When being coupled with any tumor drug, the recombinant fusion protein can form a brand-new and bio-innovative targeting pharmaceutical composition. The pharmaceutical composition shows solid tumor-specific targeting property and can rapidly shrink the tumor body and kill tumor cells, thereby achieving a targeted clinical therapeutic effect. In the present disclosure, the fusion protein produced by *Pichia pastoris* has an expression level at the gram (g) level and shows an unexpected production cost advantage during a preparation process.

To achieve the above objective, the present disclosure provides the following technical solutions:
The present disclosure provides a recombinant fusion protein, where the recombinant fusion protein is obtained by ligating a human CBD to an N-terminal or a C-terminal of HSA.

Preferably, the recombinant fusion protein is obtained by directly ligating the human CBD to the N-terminal of HSA; the recombinant fusion protein has an amino acid sequence shown in SEQ ID NO. 1; and a nucleotide sequence encoding the recombinant fusion protein is shown in SEQ ID NO. 2.

Preferably, the recombinant fusion protein is obtained by ligating the human CBD to the C-terminal of HSA through a connecting peptide (GGGS)₂; the recombinant fusion protein has an amino acid sequence shown in SEQ ID NO. 3; and a nucleotide sequence encoding the recombinant fusion protein is shown in SEQ ID NO. 4.

Preferably, the recombinant fusion protein is obtained by ligating the human CBD to the C-terminal or the N-terminal of HSA through a connecting peptide (GGGS)₂₋₅.

Preferably, the amino acid sequence of HSA further includes a natural sequence, or a sequence after amino acid substitution and variation, or a fragment of HSA.

The present disclosure further provides a recombinant engineered host expressing the recombinant fusion protein, where the recombinant engineered host is selected from the group consisting of a yeast, a Chinese hamster ovary (CHO) cell, a bacterium, a plant cell, an insect cell, and an animal cell;
the host is preferably the yeast, more preferably *Pichia pastoris.*

The present disclosure further provides a construction method of the recombinant engineered host, including the following steps: transferring a coding gene of the recombinant fusion protein into a host through gene vector expression, virus vector expression, or a transgenic method; where the host is selected from the group consisting of a yeast, a CHO cell, bacterium, a plant cell, an insect cell, and an animal cell; the host is preferably the yeast, more preferably *Pichia pastoris.*

The present disclosure further provides a pharmaceutical composition based on the recombinant fusion protein, where the pharmaceutical composition is coupled with a chemotherapy drug or a tumor cytotoxic protein using the recombinant fusion protein as a matrix.

The present disclosure further provides use of the pharmaceutical composition in preparation of a drug for specific targeted treatment of a solid tumor.

The present disclosure further provides a drug for specific targeted treatment of a solid tumor, where an active ingredient of the drug is the pharmaceutical composition.

The present disclosure further provides a drug for specific targeted treatment of a solid tumor, where an active ingredient of the drug is the pharmaceutical composition.

Compared with the prior art, the present disclosure has the beneficial effects as follows: the present disclosure provides a recombinant fusion protein, where the recombinant fusion protein is obtained by ligating a human CBD to a C-terminal or an N-terminal of HSA. In the present disclosure, the recombinant fusion protein can be used as a broad-spectrum new solid tumor-specific targeting drug matrix, and can be coupled with any tumor drug to form a brand-new and bio-innovative targeting pharmaceutical composition. The matrix is coupled with tumor therapeutic drugs, especially small-molecule cytotoxic compounds and cytotoxic proteins, thereby avoiding the serious hematological toxicity faced when small-molecule compounds (such as chemotherapy drugs) and cytotoxic proteins that require high dosages and high-frequency medications are applied alone. Meanwhile, this mechanism can also significantly increase a half-life of these tumor drugs in the human body and achieve better tumor treatment effects. The present disclosure further provides a construction method of the long-acting and broad-spectrum tumor-specific targeting drug matrix. Since the recombinant fusion protein is secreted, it can be combined with antibodies that specifically recognize anti-HSA, and can also be combined with antibodies that specifically recognize human CBDs. In an example, a yeast is used to express the recombinant fusion protein, especially a ton-scale fermentation process of *Pichia pastoris* engineered strains and a method for separation and purification of recombinant fusion protein are established. An expression level of the protein in *Pichia pastoris* in a 1,000 L fermenter is not less than 1 g/L, purity after purification can reach 99%, and an endotoxin content is less than or equal to 0.5 EU/mg. An expression system of *Pichia pastoris* can produce a long-acting and broad-spectrum recombinant fusion protein targeting matrix for tumor therapy, and the recombinant protein drug prepared through this system is completely suitable for clinical applications. A residual amount of host protein in a stock solution of the innovative drug developed in the present disclosure also meets requirements of the Third Part of the Chinese Pharmacopoeia (2015 edition).

### BRIEF DESCRIPTION OF THE DRAWINGS

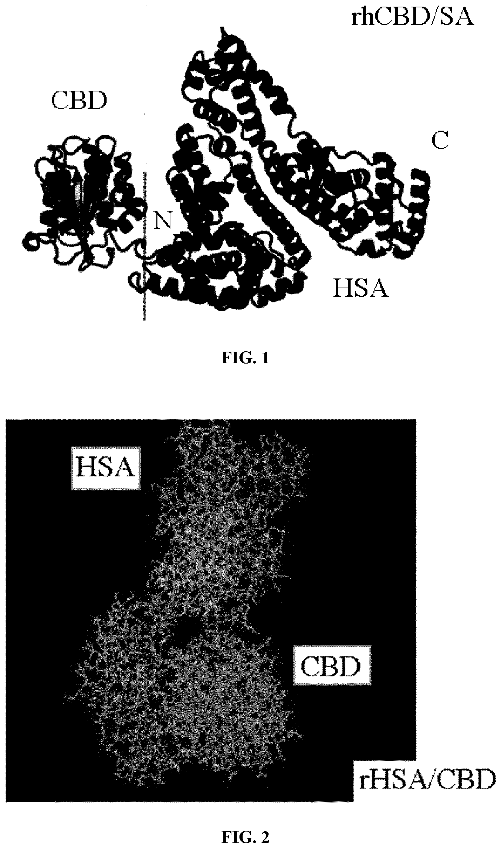

FIG. 1 shows that when the CBD is at an N-terminal of an HSA mature peptide and no connecting peptide is confirmed by RaptorX analysis; the results of rhCBD/SA molecular structure analysis show that it does not affect the independence of CBD at all, that is, the CBD shows an ability to bind to vascular collagen.
FIG. 2 shows that when the CBD is at a C-terminal of an HSA mature peptide and no connecting peptide is confirmed by RaptorX analysis; the results of rHSA/CBD molecular structure analysis show that it may affect the independence of CBD at all, that is, the ability of CBD to bind to vascular collagen may be affected.

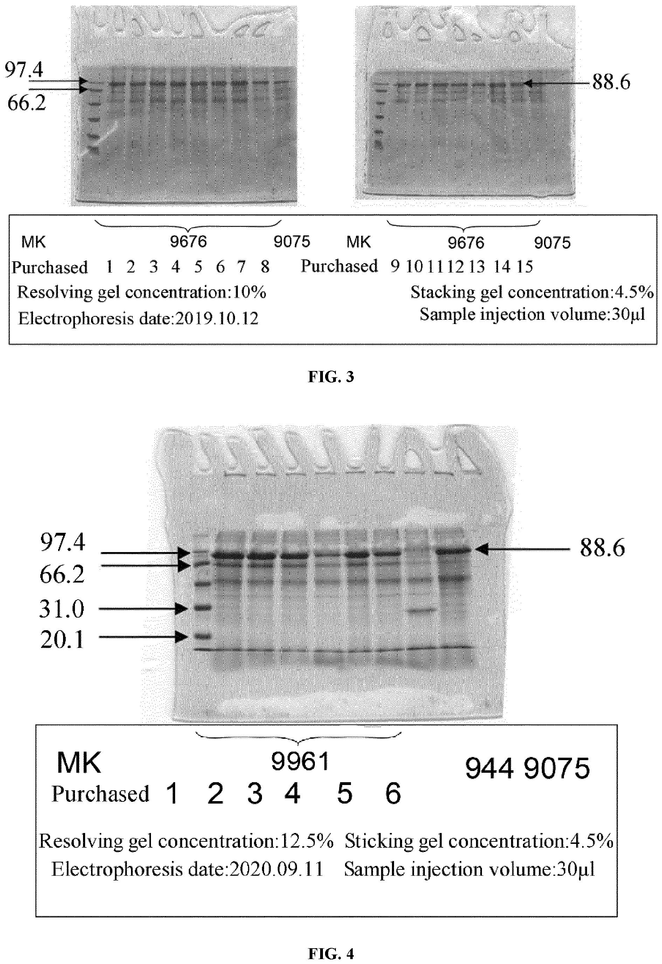

FIG. 3 shows an experimental screening result of expression engineered strains of *Pichia pastoris* for a recombinant HSA/CBD fusion protein (rHSA/CBD, code: 9676, molecular weight: 88 kD), one of the recombinant fusion proteins in screening long-acting and broad-spectrum tumor treatment targeting matrix; wherein the code 9075 in the figure is a recombinant HSA/growth hormone (GH) fusion protein (rHSA/GH, molecular weight: 88.6 kD) as a positive control.
FIG. 4 shows an experimental screening result of expression engineered strains of *Pichia pastoris* for a recombinant CBD/serum albumin (SA) fusion protein (rhCBD/SA, code: 9961, molecular weight: 88.1 kD), one of the recombinant fusion proteins in screening long-acting and broad-spectrum tumor treatment targeting matrix; where 944 in the figure is the expression of light chain of fully human monoclonal antibody in yeast engineering bacteria (code: 944, molecular weight: 23.7 kD), and code 9075 represents recombinant HSA/GH fusion protein (rHSA/GH, molecular weight: 88.6 kD) as a positive control.

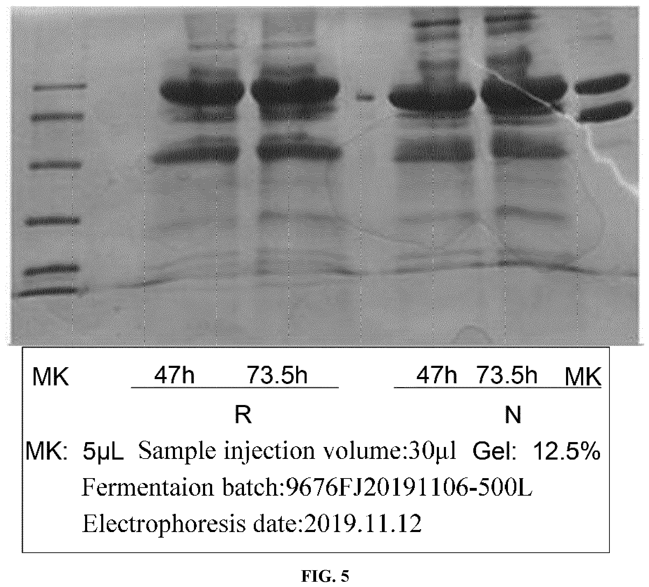

FIG. 5 shows results of the expression of a target protein (rHSA/CBD) in a supernatant at different times when the recombinant fusion protein for long-acting and broad-spectrum tumor treatment targeting matrix is fermented in a 500 L fermenter by *Pichia pastoris* engineered bacteria.

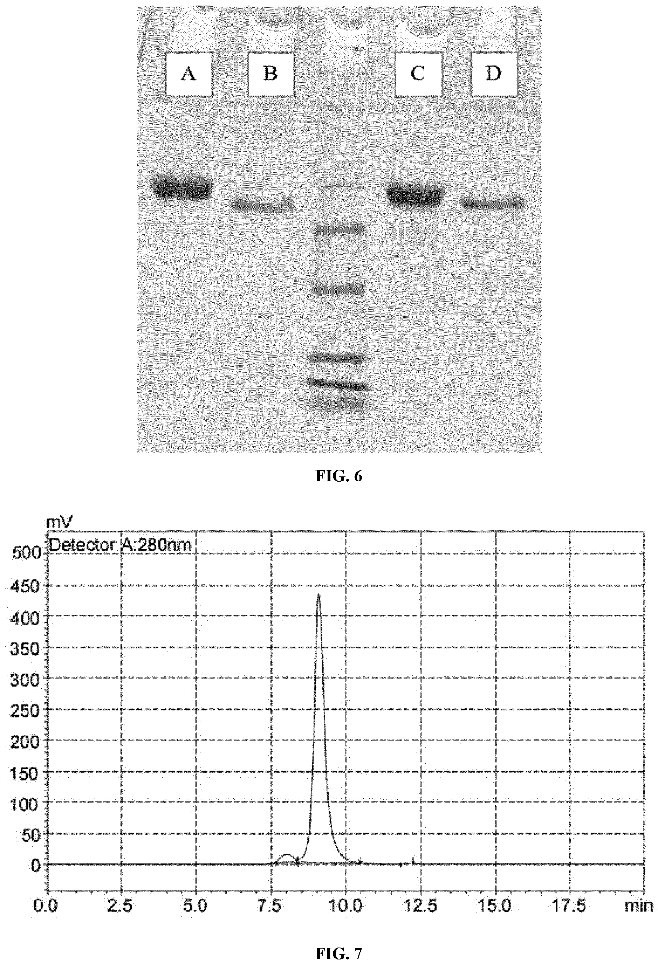

FIG. 6 shows an SDS-PAGE protein electrophoresis diagram of a purified stock solution under Reducing (samples A and B) and Non-Reducing (samples C and D) conditions, where the purified stock solution is obtained based on that in the recombinant fusion protein of long-acting and broad-spectrum tumor treatment targeting matrix, there is a connecting peptide (A and C) between the C-terminal of HSA, or a connecting peptide between the N-terminal of HSA (B and D); and standard molecular weights are in a middle band (from top to bottom: 97 kD, 66 kD, 45 kD, 35 kD, 24 kD, 14 kD).
FIG. 7 shows purity of the stock solution of the recombinant fusion protein (rHSA/CBD) for long-acting and broad-spectrum tumor treatment targeting matrix (CBD is located at the C-terminal of HSA, with a connecting peptide) determined by size exclusion chromatography-high-performance liquid chromatography (SEC-HPLC).

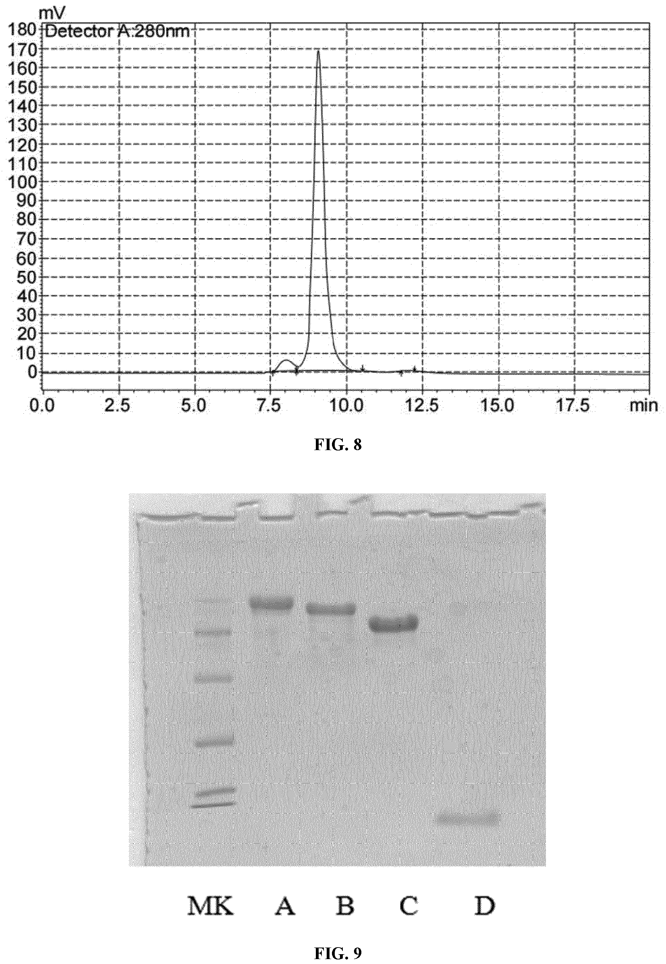

FIG. 8 shows purity of the stock solution of the recombinant fusion protein (rhCBD/SA) for long-acting and broad-spectrum tumor treatment targeting matrix (CBD is located at the N-terminal of HSA, without a connecting peptide) determined by size exclusion chromatography-high-performance liquid chromatography (SEC-HPLC).
FIG. 9 shows an SDS-PAGE electrophoretogram of an rHSA/CBD-EGF protein (molecular weight about 95 kD) formed after the recombinant fusion protein is coupled with EGF, as well as the rHSA/CBD (molecular weight about 88.2 kD), rHSA/EGF (molecular weight about 72.5 kD), and rhEGF (molecular weight 6.2 kD); and

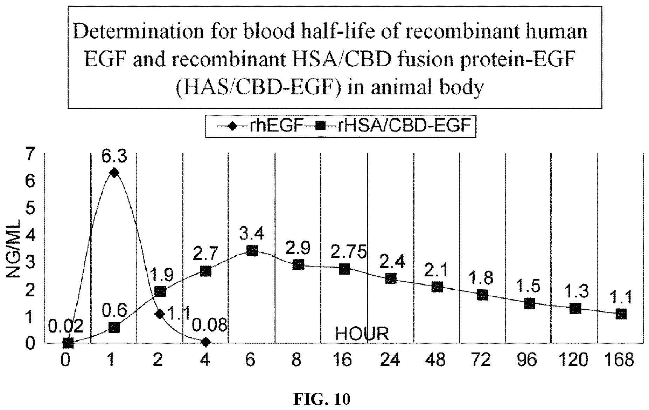

FIG. 10 shows half-life measurement results of a pharmaceutical composition (rHSA/CBD-EGF) formed after the recombinant fusion protein is coupled with EGF in rats.

### DETAILED DESCRIPTION OF THE EMBODIMENTS

The present disclosure will be further described below with reference to the examples and accompanying drawings.

The present disclosure provides a recombinant fusion protein, where the recombinant fusion protein is obtained by ligating a human CBD to an N-terminal or a C-terminal of HSA.

In the present disclosure, HSA and the human CBD can be ligated in various ways: connecting peptide (GGGS)₂₋₅ and direct ligation. The amino acid sequence of the recombinant fusion protein formed by directly ligating human CBD at the N-terminal of HSA is preferably shown in SEQ ID NO. 1, and the nucleotide sequence encoding the recombinant fusion protein is preferably shown in SEQ ID NO. 2. The C-terminal of HSA is ligated to the human CBD through a connecting peptide (GGGS)₂, the amino acid sequence of the formed recombinant fusion protein is preferably shown in SEQ ID NO. 3, and the nucleotide sequence encoding the recombinant fusion protein is preferably shown in SEQ ID NO. 4. Both the C-terminal and N-terminal of HSA can be ligated with human CBD through the connecting peptide (GGGS)₂₋₅.

In the present disclosure, RaptorX is preferably used to predict the structure and function of gene fusion: when CBD is at the N-terminal of HSA, the molecular structure of rhCBD/SA shows that it does not affect the independence of CBD at all. That is, the CBD has the ability to bind to vascular collagen (FIG. 1). Thus, the CBD can be directly ligated to the N-terminal of the HSA mature peptide, and no connecting peptide is required. When CBD is at the C-terminal of HSA, computer simulations show that some regions of CBD are directly ligated with some regions of HSA, causing interaction (FIG. 2). This may affect the ability of the CBD to bind to vascular collagen. In view of this, during constructing a recombinant fusion protein when the CBD is at the C-terminal of HSA, a connecting peptide (GGGS)₂ is required between HSA and CBD. In this way, the formation and construction of the CBD protein in the molecular structure is ensured, while not affecting the ability and function of combining with the collagen on the inner wall of the blood vessel of the tumor body.

In the present disclosure, a gene sequence of HSA has been registered in GeneBank with the accession number AY728024, and an amino acid sequence of its mature peptide is registered in the GeneBank with the registration number AY728024. The amino acid sequence of HSA includes preferably a natural sequence, or a sequence after amino acid substitution and variation, or a fragment of HSA.

The present disclosure further provides a recombinant engineered host expressing the recombinant fusion protein, where the recombinant engineered host is selected from the group consisting of a yeast, a Chinese hamster ovary (CHO) cell, a bacterium, a plant cell, an insect cell, and an animal cell. The recombinant engineered host is preferably yeast, more preferably *Pichia pastoris.*

The present disclosure further provides a construction method of the recombinant engineered host, including the following steps: transferring a gene of the recombinant fusion protein into a host through plasmid DNA vector expression, virus vector expression, or a transgenic method; where the host is selected from the group consisting of the yeast, the CHO cell, the bacterium, the plant cell, the insect cell, and the animal cell. Preferably, the recombinant fusion protein is recombined by genetic engineering technology and expressed in engineered host cells. In an example, the recombinant fusion protein is preferably expressed by transforming and transfecting or infecting host cells with a vector plasmid; more preferably, a yeast expression vector is used to transform *Pichia pastoris,* and the recombinant fusion protein is secreted into a liquid medium. A host of the yeast expression vector is preferably a Pasteur strain of *Pichia.*

The present disclosure further provides a method for large-scale and high-density expression and production of recombinant fusion proteins (rHSA/CBD and rhCBD/SA) with two structures, including the following steps: a), conducting construction and expression of two vector plasmids; b), conducting transformation and preparation of *Pichia pastoris* expression engineered bacteria; c), conducting screening of recombinant yeast engineered bacteria; and d), conducting large-scale and high-density expression to produce recombinant fusion proteins with two molecular structures (rhCBD/SA at the N-terminal; rHSA/CBD at the C-terminal).

In the present disclosure, the construction and expression of the vector plasmids preferably include: synthesizing and amplifying an *HSA* gene by using a total RNA extracted from human liver as a template through a reverse transcription-polymerase chain polymerization (RT-PCR); separating and purifying an HSA DNA fragment by electrophoresis, and cloning the fragment into a yeast expression vector to obtain a pYZ-HSA recombinant plasmid; artificially synthesizing a CBD nucleotide sequence by DNA sequence synthesis method; and digesting synthetic human CBD and pYZ-HSA recombinant plasmid with restriction enzymes, and ligating the synthetic human CBD to the pYZ-HSA recombinant plasmid to obtain pYZ-hCBD/SA and rHSA/CBD recombinant plasmids.

In the present disclosure, the transformation and preparation of the *Pichia pastoris* expression engineered bacteria preferably include: treating the pYZ-hCBD/SA and rHSA/CBD recombinant plasmids separately with a Pme I restriction endonuclease, transforming into *Pichia pastoris* X33 competent cells, and inoculating transformed yeast cells on a YPD plate medium containing Zeocin antibiotics for culture.

In the present disclosure, the recombinant yeast engineered bacteria obtained after the culture are screened. The screening preferably includes: cultivating several yeast colonies containing a gene to be expressed in a basic culture solution containing Zeocin antibiotics, buffer, and glycerol; after cultivating for a period of time, collecting bacteria by centrifugation, and resuspending the bacteria in a same basic culture solution without glycerol and containing 0.5% methanol; adding 100% methanol every 24 h to a final concentration of 0.5%, collecting culture supernatants at different time points, and screening recombinant yeast engineered strains expressing specific proteins.

In the present disclosure, the large-scale and high-density expression and production of recombinant fusion proteins with two molecular structures preferably includes: taking a tube of engineered bacteria seed bank (selected recombinant yeast engineered strains that express specific proteins), and starting small-scale culture in a shaker using 500 L and 1000 L biofermentation tank systems; amplifying the culture to a first-level seed tank, a second-level seed tank, and entering the culture mixture to production fermentation tank; after culturing the culture until the glycerin in the tank is exhausted, the dissolved oxygen returns to the bottom limit and then rises again, starting the glycerin feeding; after the feeding is completed and the dissolved oxygen rises again, starting methanol feeding, entering the process to the stage of induction and recombinant protein production, and maintaining the methanol feeding for 72 h; and obtaining a fermentation supernatant with a yield of not less than 3 g/L.

In the present disclosure, after the recombinant fusion protein is obtained, purification is preferably conducted, more preferably high-density fermentation of engineered yeast bacteria is conducted. The soluble recombinant fusion protein with a complete molecular structure that is directly secreted into the fermentation supernatant is successively passed through the following 3-step enrichment and purification production process: (1) Capto-MMC: first-stage purification initiated by a Capto-MMC packing medium of GE Company in the United States; (2) hydrophobic medium chromatography column: Phenyle, Butyle or Oycto Sepharose^{™} High Performance; (3) purification: anion exchange chromatography, cation exchange chromatography, or Capto-DEAE purification separation chromatography.

In the present disclosure, the first step of column chromatography is conducted by using a composite novel weak cation exchange chromatography Capto-MMC chromatography of GE Company of the United States. The expressed supernatant has complex components and salts of a certain concentration. The Capto-MMC chromatography can withstand higher flow rates and high binding capacity and is not demanding on a salt concentration in the fermentation supernatant. For Capto-MMC chromatography, a 10-to-100 mM NaAc-HAC buffer solution having a pH value of 4.5 to 6.5 can be used. The elution buffer can be a 10-to-100 mM phosphate buffer containing 0.1 to 0.5 M NH₄Cl or other high-salt solutions having a pH value of 4.5 to 8.5. Alternatively, various concentrations of organic solvents can be used. Optionally, a certain amount of amino acid such as glycine or cysteine is added to the buffer to maintain the stability of the CBD recombinant fusion protein.

After the purification, purity of the obtained recombinant fusion protein is not less than 95%, meeting a pharmaceutical grade for raw material drug (stock solution). The target protein in the elution has high purity and considerable recovery rate. In the present disclosure, the purification method further includes preferably conventional purification methods such as chromatography (Sephacryl S-200 HR) or membrane dialysis concentration so as to obtain a high-concentration stock solution for the production of finished clinical preparations.

The present disclosure further provides a pharmaceutical composition based on the recombinant fusion protein, where the pharmaceutical composition is coupled with a chemotherapy drug or a tumor cytotoxic protein using the recombinant fusion protein as a matrix. In the present disclosure, the recombinant fusion protein can be coupled with any tumor drug, including small-molecule compounds (chemotherapy drugs) or cytotoxic proteins to form a brand-new and bio-innovative targeting pharmaceutical composition. There is no special limitation on type and source of the small-molecular compound, which is preferably selected from the group consisting of a platinum drug, a nitrosourea drug, an antifolate drug, an antipyrimidine drug, a vinblastine drug, a taxane drug, a camptothecin drug, an anthracycline drug, a drug that disrupts a structure and a function of a DNA, a drug that is intercalated into a DNA and interferes with transcription of the DNA, and a drug that affects protein synthesis. All drugs used for treating tumors can be coupled by the recombinant fusion protein as a matrix to form a brand-new specific targeting drug for tumor treatment.

In the present disclosure, the tumor cytotoxic protein may preferably include: ricin, diphtheria toxin, trichosanthin apoptin (also known as VP3 protein), Pseudomonas aeruginosa exotoxin, an inhibitor of apoptosis protein, human epidermal growth factor, gonadotropin-releasing hormone, and deoxyribonuclease II. All cytotoxic proteins can be coupled by the recombinant fusion protein as a matrix to form a brand-new specific targeting drug for tumor treatment.

In the present disclosure, the pharmaceutical composition (taking human EGF coupled to rHSA/CBD as an example) has a longer drug metabolism half-life in animals or humans than the original *in vivo* half-life of CBD proteins or chemotherapy drugs or toxic proteins, showing long-term effect. The half-life of the pharmaceutical composition is at least 10 times higher than the *in vivo* half-life of tumor chemotherapy drugs or cytotoxic proteins. When using a drug for treating tumor indications prepared by the pharmaceutical composition, the drug should be administered once every 1 week, 2 weeks, 3 weeks, or 4 weeks. Alternatively, the drug is administered less frequently than when the chemotherapy drug or cytotoxic protein is used alone.

In the present disclosure, after entering the human blood, the pharmaceutical composition utilizes albumin's non-specific targeting tumor aggregation function. The pharmaceutical composition utilizes the CBD fused with albumin to combine with the collagen in the microvessels of the tumor body and is specifically targeted and fixed on the inner wall of the abundant microvessels of the tumor body. The pharmaceutical composition achieves specific targeting and efficient killing of tumor cells by means of chemical drugs or cytotoxic proteins for tumor treatment that are coupled to the recombinant fusion protein, thereby causing rapid tumor shrinkage and inhibiting tumor growth clinically.

The present disclosure further provides use of the pharmaceutical composition in preparation of a drug for specific targeted treatment of a solid tumor.

The present disclosure further provides a drug for specific targeted treatment of a solid tumor, where an active ingredient of the drug is the pharmaceutical composition.

Embodiments of the present disclosure will be described in detail below with reference to specific examples, but those skilled in the art will understand that the following examples are only used to illustrate the present disclosure and should not be regarded as limiting the scope of the present disclosure. If no specific conditions are specified in the examples, the examples will be conducted according to conventional conditions or the conditions recommended by the manufacturer. If the reagents or instruments used do not indicate the manufacturer, they are all conventional products that can be commercially available.

### Example 1 Gene expression of HSA and construction of carrier plasmid

HSA gene was synthesized and amplified by RT-PCR using a total RNA extracted from human liver as a template. 5 µg of the total RNA synthesized a corresponding DNA strand under the action of reverse transcriptase (purchased from GIBCO/BRL company), and the oligonucleotide primer was 18 T+lN (N is a random nucleotide). The reaction conditions were 45°C for 20 mins, then 55°C for 40 mins.

The oligonucleotide primer sequences were: 5'-GAATTCATGAAGTGGGTAACCTTTATTTCC-3' (SEQ ID NO. 5) and 5'-GAATTCTTATAAGCCTAAGGCAGCTTGACTTGC-3' (SEQ ID NO. 6).

Two EcoRI recognition sites were added to the two ends of the HSA gene and cloned into an expression vector. The PCR reaction conditions were: 94°C, 4 min, and the DNA encoding HSA was further amplified for 35 cycles: 94°C, 30 s; 58°C, 30 s; 72°C, 2 mins and 30 s. At the end of the cycle reaction, an extended reaction at 72°C for 10 mins was conducted. Gel electrophoresis showed that a PCR amplification product was about 1,850 bp in length. The PCR product was inserted into a PCRII vector (Invitrogen Company) by TA cloning method. A resulting plasmid was named PCR-HSA. DNA sequencing results showed that it was identical to the published DNA sequence of HSA (deposited in GeneBank, USA, registration number: AY728024).

The PCR product was digested with EcoRI restriction enzyme, the HSA DNA fragment was separated and purified by electrophoresis, and cloned into a yeast expression vector (pYZ vector, from Tianjin PuYing Biotechnology Co., Ltd.). After transformation into DH5a competent cells, positive single clones were selected on low-salt LB-agar plate containing antibiotic Zeocin, and a plasmid inserted into the HSA gene was identified as pYZ-HSA (as shown in FIG. 1, pYZ-HSA recombinant plasmid EcoRI & XhoI was digested and identified, and the target band size was about 1,850 bp, which was consistent with the expected target band size. Sequencing was conducted to confirm forward and reverse directions).

### Example 2 Expression of recombinant fusion protein gene (rhCBD/SA) and construction of carrier plasmid for long-acting and broad-spectrum tumor treatment targeting matrix

According to the known literature analysis and the complete amino acid sequence of vWF (von Willebrand factor [Homo sapiens] GenBank# AAB59458.1 von Willebrand factor [Homo sapiens]), the human vWF A3 domain was Cys1670-Gly1874 (peptide segment 907 to 1111 of human vWF mature peptide amino acids), which was CBD, with a molecular weight of 20 kD.

The rhCBD/SA mature peptide was synthesized according to the amino acid sequence shown in SEQ ID NO. 1: the CBD mature peptide was directly connected to the N-terminal of HSA, and there was no connecting peptide between them.

Therefore, according to the amino acid sequence of rhCBD/SA mature peptide, the nucleotide sequence of CBD mature peptide was artificially synthesized by DNA sequence synthesis. During artificially synthesizing, an Xho I restriction site could be added to the N-terminal, and a HSA sequence could be added to an Ale I restriction site at the C-terminal. These two enzymes could be used to digest the synthesized target gene *CBD* and the *HSA* gene sequence in pYZ-HSA to form recombinant plasmids, respectively. For secreted peptides, yeast α-Factor could be used to secrete the recombinant sequence of the target gene *CBD* and *HSA* gene. The digested target gene *CBD* and HSA recombinant DNA plasmid were mixed in a certain ratio, a ligation buffer and ligase were added, and ligation was conducted at 22°C for 1 h. A ligation product was transformed into DH5α competent cells: the ligation product was added to DH5α competent cells, treated in ice bath for 30 min, heat-shocked at 42°C for 90 s, then treated in ice bath for 2 min, added into a culture medium and placed in a shaker, centrifuged at 150 rpm for 45 min and 12000 rpm for 1 min, and spread on a plate. Sequencing verification: a single clone was selected and subjected to expanded culture; a small amount of plasmid was extracted, identified by enzyme digestion; a plasmid with the same size as the target fragment and obtained by enzyme digestion was selected, and confirmed by sequencing. The sequencing primers were forward P1 and reverse P9. The plasmid confirmed by the full sequencing was named pYZ-rhCBD/SA.

P1: 5'-CTGGTTCCAATTGACAAGC-3' (located in the promoter sequence, SEQ ID NO. 7);

P9: 5'-GACTCATCAGCAACACATG-3' (located in HSA sequence, SEQ ID NO. 8). Based on this, a nucleotide DNA sequence (SEQ ID NO.2) of rhCBD/SA was synthesized.

### Example 3 Expression of recombinant fusion protein gene (rHSA/CBD) and construction of carrier plasmid for long-acting and broad-spectrum tumor treatment targeting matrix

The N-terminal of the CBD was ligated to the C-terminal of HSA, and a rigid connecting peptide (GGGSGGGS) was provided between them to ensure that the CBD at the C-terminal of the fused HSA did not occur in the state shown in FIG. 2 that there was no connecting peptide between the recombinant fusion protein. To this end, a molecular structure of novel tumor-specific targeting drug matrix shown in SEQ ID NO. 3 was artificially synthesized. For the gene synthesis method, reference can be made to that in Example 2, and a finally obtained rHSA/CBD had a nucleotide DNA sequence shown in SEQ ID NO. 4.

### Example 4 Transformation and preparation of Pichia pastoris expression engineered bacteria

*Pichia pastoris* strain X33 was inoculated into a 50 ml centrifuge tube containing 5 ml YPD medium, and cultured overnight at 30°C and 250 rpm. The next day, 0.2 ml of overnight culture was transferred into 500 ml of YPD culture medium and placed in a 2 L Erlenmeyer flask. The cells were subjected to rotation culture at 30°C for 2 h to 3 h to make a cell density reach OD₆₀₀=1.3 to 1.5. The yeast was collected by centrifugation, resuspended in 500 ml ice-cold sterile water and washed twice. Afterwards, the yeast was suspended in 20 ml ice-cold 1 M Sortbitol solution and washed once.

The pYZ-rhCBD/SA plasmid DNA or pYZ-HSA/CBD constructed in Examples 2 and 3 were treated with Pme I restriction endonuclease to form linearized plasmid molecules. 5 µg of linearized plasmid DNA was mixed with 80 µl of treated yeast and placed in a 0.2 cm thick electrode cup and placed on the electroporator. The electric pulse was conducted at a voltage of 7,500 V/CM and an electrode interval of 5-10 (ms). Immediately after the electric shock treatment, 1 ml of ice-cold 1 M sorbitol solution was added to the yeast, and then transferred into a 15 ml test tube. The transformed yeast was placed in an incubator at 30°C for 2 h, and then inoculated and spread on a YPD plate medium containing Zeocin antibiotic. The clones grown after resistance selection were identified for gene insertion by molecular biology methods. Protein expression and secretion were detected by SDS-PAGE or Western blot with specific antibodies.

### Example 5 Screening of Pichia pastoris engineered bacteria expressing recombinant fusion protein gene

Several yeast colonies containing a gene to be expressed were cultured in basic culture medium containing Zeocin antibiotic, buffer, and glycerol. The cells were cultured at a speed of 300 rpm until a cell density reached OD_{600 nm}=2-6. An obtained culture was centrifuged at 1,500 rpm for 15 min, and the collected bacteria were resuspended in a same basic culture medium without glycerol and with 0.5% methanol until the cell density reached OD_{600 nm}=1.0, and the culture was continued. Under the induction of methanol, the yeast began to express the foreign protein under the action of the promoter. 100% methanol was added every 24 h to a final concentration of 0.5%. Culture supernatants were collected at different time points. The screening results were initially determined by SDS-PAGE denaturing polyacrylamide gel electrophoresis. Based on FIG. 3: recombinant HSA/CBD recombinant fusion protein (rHSA/CBD, code: 9676, molecular weight: 88 kD) or/and FIG. 4: recombinant human CBD/SA recombinant fusion protein (rhCBD/SA, code: 9961, molecular weight: 88.1 kD), the recombinant yeast engineered strains expressing specific proteins of interest were selected. Recombinant HSA/GH fusion protein (rHSA/GH, code: 9075; molecular weight: 88.5 kD) and light chain of fully human monoclonal antibody expressed by yeast engineered bacteria (code: 944, molecular weight: 23.7 kD) were used as positive controls for screening, respectively.

### Example 6 Process for large-scale and high-density fermentation for expression and production of recombinant fusion protein

In the present disclosure, the expression of the amino acid sequences of rhCBD/SA and rHSA/CBD mature peptides was conducted using a 500 L or 1000 L (ton-level) biological fermenter system. An engineered bacteria seed bank was prepared using the recombinant yeast engineered bacteria according to the developed process. A seed tube of the working seed bank of engineering bacteria was inoculated into a triangular flask, starting from a small-scale shaker culture, and then expanded to a first-level seed tank, a second-level seed tank, and then a production fermentation tank (1 ton by volume). After cultivating until the glycerin in the tank was exhausted, and the dissolved oxygen returned to the bottom limit and then rose again, glycerin feeding was started. When the glycerol feeding was completed and the dissolved oxygen rose again, methanol feeding was started, and the induction and recombinant protein production phases were started, and the methanol feeding was maintained for 72 h. Samples were taken at different culture time points to test the expression of the recombinant protein. The content of the secreted protein in the cells and in the culture medium was analyzed by SDS-PAGE, and the expression level and purity were monitored in each step. The results showed that the expression level of the recombinant fusion protein in the fermentation broth was 3-10 g/L. FIG. 5 illustrated the results of 1 fermentation batch: 9676FJ20191106-500L using a 500 L fermenter. SDS-PAGE was divided into electrophoresis under reducing SDS-PAGE (Reducing) and non-reducing SDS-PAGE (Non-Reducing) conditions. The difference between reducing (R) and non-reducing (N) SDS-PAGE is to add or not add a reducing agent (such as DTT or 2-mercaptoethanol) during sample processing.

### Example 7 Processes for isolation and purification of recombinant fusion protein secreted by yeast engineered bacteria

Both rHSA/CBD and rhCBD/SA recombinant fusion proteins could be directly secreted into the supernatant after yeast engineered bacteria fermentation. After the supernatant containing the secreted recombinant fusion protein was separated from the bacterial cells by continuous flow centrifugation, column chromatography purification was first conducted by a recombinant HSA recombinant fusion protein purification process disclosed in patent ZL 200810089645.1. The fermentation supernatant of yeast engineered bacteria (code: 9676FJ20191106-500L) could be directly purified according to the following separation and purification procedures:
a. Primary affinity chromatography column: Capto-MMC;
   Equilibration buffer: 10 mM NaAc-HAc, 0.5 mM cysteine, 2% glycine, pH 5.0;
   Purity wash buffer: 10 mM PB, 2 mM cysteine, 2% glycine, pH 6.0;
   Elution buffer: 20 mM PB, 2 mM cysteine, 2% glycine, 1.0 M NH4Cl, pH 7.5.
b. The comparison and screening were conducted by hydrophobic fillers with butyl-, phenyl-, or octyl-, and 20-100 mM Tris-HCl or phosphate buffer was selected, at a pH value of 5.0 to 9.0. A 1-2 M NH₄Cl or 1-2 M (NH₄)₂SO₄ buffer was used as the equilibration buffer, and a buffer without NH₄Cl or (NH₄)₂SO₄ was used as an elution buffer. A certain amount of glycine or cysteine could also be added to the buffer to maintain protein stability. An elution peak of the Capto-MMC column was diluted several times with 20-100 mM Tris-HCl or phosphate buffer containing 1-2 M NH₄Cl or 1-2 M (NH₄)₂SO₄ at a pH of 5.0 to 9.0. Alternatively, high-concentration NH₄Cl or (NH₄)₂SO₄ solution was directly added to the elution peak of the Capto-MMC column to a concentration of 1-2 M NH₄Cl or 1-2 M (NH₄)₂SO₄, and the sample was loaded on the hydrophobic column. The column was rinsed with the equilibration buffer, then eluted with the elution buffer, and eluted peaks were collected. Impurities such as polymers, sugars, lipids, degraded proteins, and pigments that might exist could be further removed through the hydrophobic column, and the purity of the target protein was further improved.

The samples purified by hydrophobic chromatography were also directly repurified from anion exchange columns such as Q or DEAE in consideration of the production process. Ion exchange chromatography could further purify the target protein through a different separation mechanism than the hydrophobic chromatography. 20-100 mM Tris-HCl or phosphate buffer with a pH range of 6.0 to 9.0 could be used as the equilibrium buffer, or a certain amount of glycine or cysteine could be added to the buffer to maintain protein stability. The concentration of 200-1000 mM NaCl or NH₄Cl can be added to the equilibration buffer as the elution buffer. The samples after anion exchange chromatography could usually achieve a purity of not less than 95%.
c. Ion purification separation chromatography column: Capto-DEAETM Fast Flow;
Equilibration buffer: 20 mM Tris, 3 mM cysteine, 2% glycine, pH 7.2;
Purity wash buffer: 20 mM Tris, 180 mM NaCl, 10 mM cysteine, 5% glycine, pH 7.2;
Elution buffer: 20 mM Tris, 800 mM NaCl, 10 mM cysteine, 5% glycine, pH 7.2.

The elution peaks collected after ion exchange chromatography could be directly loaded on Sephacryl, Superdex, or Sephadex and other gel chromatography columns for desalting and liquid replacement. In the gel chromatography, 5-50 mM phosphate buffer (PB) was used as the equilibrium buffer, and a certain amount of glycine or cysteine could also be added to the buffer to maintain protein stability. The buffer system of the target protein CBD-HSA obtained under anion-exchange chromatography conditions could be changed to 5-10 mM phosphate buffer solution, and could be directly used in the production of preparations without using other means such as dialysis or ultrafiltration. This reduced possible contamination, protein denaturation, degradation, and aggregation losses during processing steps.
or c. Chromatographic column: Sephacryl S-200 HR;
Equilibration buffer: 20 mM PB, 5% glycine, pH 7.2.

The entire purification process had a reasonable sequence, easy operation, and easy industrial scale-up production, and the final product had a single component of up to 99% (≥95%). The aggregates, dimers, and related proteins of the target protein were calculated as the target protein, and the residual amount of the host protein was less than 0.05%. In the present disclosure, the separation and purification production process that could be used in large-scale production and preparation of recombinant fusion proteins was formed, and a final purity reached an unexpected high purification effect of not less than 99%. FIG. 6 showed an SDS-PAGE protein electrophoresis diagram of a purified stock solution under Reducing (samples A and B) and Non-Reducing (samples C and D) conditions, where the purified stock solution is obtained based on that in the recombinant fusion protein of long-acting and broad-spectrum tumor treatment targeting matrix, there is a connecting peptide (A and C) between the C-terminal of HSA, or a connecting peptide between the N-terminal of HSA (B and D); and standard molecular weights are in a middle band (from top to bottom: 97 kD, 66 kD, 45 kD, 35 kD, 24 kD, 14 kD).

### Example 8 HPLC for determination of purity of recombinant fusion protein

A Shimadzu LC-10Avp Plus high-performance liquid chromatograph was used, and the chromatographic column was: TSK-GEL G2000SWXL 7.8×300 mm column from TOSOH Company. A 50 mM PB-0.1 M NaCl buffer solution at pH=7.0 was used as a mobile phase, flow rate: 0.8 ml/min, detection wavelength: 280 nm.

After the optimized separation and purification process established by the above procedure, a purity of the recombinant fusion protein was detected by SEC-HPLC. The results showed that the purity of the rHSA/CBD sample was about 99.62% (FIG. 7), and the purity of the rhCBD/SA sample was about 99.04% (FIG. 8). The protein content related to the target protein (target protein aggregates and target protein degradation products) was less than 1%.

### Example 9 Residue detection of yeast host protein of recombinant fusion protein

It was detected by double-antibody sandwich enzyme-linked immunoassay. The host protein residue of the recombinant fusion protein was not higher than 0.01% of the total protein. Detection kit was: Immunoenzymetric Assay Kit for the Measurement of Pichia pastoris Host Cell Proteins (Cygnus, USA), item number: Cat# F140, detection wavelength 450/650 nm; standard solution preparation: a standard solution provided in the kit was used. The recovery assay wells were set up (duplicate wells). An assay method was based on operating instructions provided by the kit manufacturer.

The results showed that the host protein residue in the recombinant fusion protein stock solution should not be higher than 0.01% of the total protein. In the high-purity injectable recombinant fusion protein product produced by the production and preparation process of the present disclosure, the test results showed that the residual amount of the host protein was already below 0.01%. That is, the purity of the target protein had reached 99.99%.

### Example 10 Coupling of recombinant fusion protein to small-molecular compound

The recombinant fusion protein was diluted with 10 mM PBS containing 2 mM EDTA. 4 times the equivalent molecular mole of Traut's reagent (a product of Traut Reagent Abcam Company, Cat# ab145615, the main component is 2-iminosulfane hydrochloride, CAS#: 4781-83-3; Thermo's Traut's reagent was Cat# 26101; Pierce also sells Traut's reagents) was dissolved in the same PBS containing 2 mM EDTA, added to the protein solution, and incubated at room temperature in the dark for 1 h. Traut reagent was removed using a Zeba spin desalting column (Thermo Fisher). The protein was added to the protein solution free of Traut reagent, and dissolved in Sigma-Aldrich pharmaceutical-grade doxorubicin (Al doxorubicin) in 10 mM sodium phosphate buffer (pH=5.9) at a molecule molar ratio of 15, or in 5-fluorouracil or other small-molecule drugs, and then incubated at room temperature in the dark for another 1 h. To stop the reaction, 20 mM L-cysteine [dissolved in PBS buffer containing 2 mM EDTA] was added. Unreacted and uncoupled doxorubicin (small-molecule compound) could be removed by centrifugation (10,000 g, 5 min). The supernatant was further purified with a Zeba spin desalting column followed by ultrafiltration with Amicon Ultra (Merck, 10 kD molecular cut-off). The concentrated protein product was quantified by Pierce's bicinchoninic acid assay (BCA) protein content assay method, the protein assay kit was purchased from Thermo Fisher, and the method of use was operated according to the kit instructions. The content of doxorubicin coupled to the recombinant fusion protein in the pharmaceutical composition was determined by measuring the absorbance at 495 nm and quantifying the extinction coefficient (L/mol/cm) of 10,650. The results showed that in the final pharmaceutical composition product after coupling, the binding rate of doxorubicin content was about 3 to 5 times of the molecular molarity of the recombinant fusion protein. The results showed that at least 2 to 5 doxorubicin drug molecules could be coupled to each recombinant fusion protein. In the same way, the test confirmed that the recombinant fusion protein could be coupled with small-molecule compounds and chemotherapy drugs. When coupled with different small molecule drugs, different innovative pharmaceutical compositions could be formed, and then used as therapeutic drugs for different clinical indications of solid tumors.

### Example 11 Production process for recombinant fusion protein coupled with rhEGF to form nanoparticles

The specific experimental method adopted the method established by Wang Shubin et al. (Chinese Oncology Clinic, 2008, 35:9, 516-519). 1 ml of a water phase (20% recombinant fusion protein saline solution) and 50 ml of an oil phase (cyclohexane, containing 2% emulsifier OP-4), stirred and mixed at 600 r/min for 1 h, and emulsified for 20 min. An emulsified product was cured with 25% glutaraldehyde toluene solution as a cross-linking agent and stirred for 2 h. rhEGF (patent: ZL200710057571.9) was covalently linked to rHSA/CBD nanoparticles (NPs) through a soluble crosslinker 1-ethyl-3-(3-dimethylaminopropyl carbodiimide) (EDC). 1 mg of the recombinant fusion protein nanocarrier was added to 1 ml of 0.1 mol/L MES solution (pH=6.0), and 0.4 mg of EDC (2 mmoL) and 0.6 mg of N-hydroxysulfosuccinimide (Sulfo-NHS) were added. After scaling up according to this ratio, reaction was conducted at room temperature for 15 min, rhEGF (100 µg) prepared by engineered yeast bacteria was added, and stirred at room temperature for 2 h. In the ligation reaction, the specific separation pharmaceutical composition EGF and the recombinant fusion protein nano-matrix carrier were effective components with a molar ratio of 1:1. FIG. 9 showed the SDS-PAGE profiles of rHSA/CBD-EGF protein (molecular weight about 95 kD), rHSA/CBD (molecular weight about 88.2 kD), and rHSA/EGF (molecular weight about 72.5 kD) after coupling.

The recombinant human EGF coupled to the recombinant fusion protein (rHSA/CBD) as a drug-matrix complex was detected with a commercially available hEGF detection ELISA kit (Abcom, Cat# ab217772). The test results showed that it was similar to the molecular structure of the recombinant HSA/EGF fusion protein (rHSA/EGF) disclosed by our company (patent ZL200710057571.9). The results showed that the coupling ratio was as predicted for the molecular molar ratio (1:1).

According to the Pharmacopoeia method, the biological activity of the rHSA/CBD-EGF complex was measured at the cellular level [2015 edition of the Chinese Pharmacopoeia Three General Rules 3528: Recombinant human epidermal growth factor biological activity assay (cell proliferation method/MTT colorimetric method)]. The results showed that when the rHSA/EGF recombinant fusion protein had the same mole number, the measured value of biological activity was similar. Measurement results were shown in Table 1.

**Table 1 Determination of EGF biological activity of samples (colorimetric method)**

| Sample name | Batch number | Molecular weight (kD) | Protein content (mg/ml) | Biological specific activity (lU/mg) |
|---|---|---|---|---|
| rhEGF Monomer | E20Y20140602 | 6.2 | 1.25 | 13.2×10⁵ |
| rHSA/EGF Recombinant HSA/EGF fusion protein | X012Y20091203 | 72.7 | 17.83 | 1.57×1⁰⁵ |
| rHSA/CBD Matrix | 9676Y191201 | 88.6 | 5 | None |
| rHSA/CBD-EGF Coupled pharmaceutical composition | 9676/EGF 1910 | 94.3 | 2 | 1.1×10⁵ |

### Example 12 Determination of half-life of recombinant fusion protein-EGF pharmaceutical composition in rats

Sprague-Dawley rats (SD, SPF grade, half male and half male) were used. The body weight of the rats was measured on the day just before administration (D-1), and the body weight range of male rats should be about 276-372 g, and that of female rats should be about 221-280 g. The test drug was the stock solution of the recombinant fusion protein-EGF complex of the present disclosure (recombinant HSA/CBD fusion protein-EGF, rHSA/CBD-EGF), specification: 2.5 mg/0.5 ml/bottle (protein concentration: 5 mg/ml). When the test drug was administered to rats, the dosage was 200 µg/kg. Each dosing volume was 300 µl. Blood was collected 1 h before administration, and 2 min, 0.5 h, 2 h, 4 h, 6 h, 8 h, 24 h, 48 h, 72 h, 96 h, 120 h, and 168 h after administration. After anesthesia with isoflurane inhalation, blood was collected from the orbital venous plexus, with a blood volume of about 0.05 ml per mouse. After collection, the blood sample was transferred into an EP tube containing EDTA, shaken well, placed in crushed ice (stored in crushed ice for no more than 2 h), and then centrifuged at 4,000 rpm for 10 min at low temperature (about 4°C) to separate the plasma, stored below -20°C for analysis. The half-life of the coupled drug rhEGF was determined to be 1-2 h, while the blood half-life of the coupled rHSA/CBD-EGF was 80-100 h. The results in FIG. 10 showed that the blood half-life of the EGF polypeptide protein monomer after being coupled to the rHSA/CBD recombinant fusion protein was increased or extended at least 30 times. Moreover, the biological activity of EGF in the coupled pharmaceutical composition did not change, confirming that the recombinant fusion protein could be used as a matrix carrier to couple protein polypeptides or toxic proteins, and the half-life of the coupled protein polypeptides was greatly extended. The results verified that the recombinant fusion protein coupled with drug molecules could greatly prolong the half-life of the drug *in vivo,* and provided technical support for the formation of innovative solid tumor-specific targeting pharmaceutical compositions.

Described above are merely preferred implementations of the present disclosure. It should be noted that a person of ordinary skill in the art may further make several improvements and modifications without departing from the principle of the present disclosure, but such improvements and modifications should be deemed as falling within the protection scope of the present disclosure.

## Claims

1. A recombinant fusion protein, wherein the recombinant fusion protein is obtained by ligating a human collagen-binding domain (CBD) to an N-terminal or a C-terminal of a human serum albumin (HSA).

2. The recombinant fusion protein according to claim 1, wherein the recombinant fusion protein is obtained by directly ligating the human CBD to the N-terminal of HSA; the recombinant fusion protein has an amino acid sequence shown in SEQ ID NO. 1; and a nucleotide sequence encoding the recombinant fusion protein is shown in SEQ ID NO. 2.

3. The recombinant fusion protein according to claim 1, wherein the recombinant fusion protein is obtained by ligating the human CBD to the C-terminal of HSA through a connecting peptide (GGGS)₂₋₅; the recombinant fusion protein has an amino acid sequence shown in SEQ ID NO. 3; and a nucleotide sequence encoding the recombinant fusion protein is shown in SEQ ID NO. 4.

4. The recombinant fusion protein according to claim 1, wherein the recombinant fusion protein is obtained by ligating the human CBD to the N-terminal of HSA through a connecting peptide (GGGS)₂₋₅.

5. The recombinant fusion protein according to any of claims 1 to 4, wherein the amino acid sequence of HSA further comprises a natural sequence, or a sequence after amino acid substitution and variation, or a fragment of HSA.

6. A recombinant engineered host expressing the recombinant fusion protein according to any of claims 1 to 5, wherein the recombinant engineered host is selected from the group consisting of a yeast, a Chinese hamster ovary (CHO) cell, a bacterium, a plant cell, an insect cell, and an animal cell.

7. The recombinant engineered host according to claim 6, wherein the recombinant engineered host is the yeast.

8. The recombinant engineered host according to claim 7, wherein the recombinant engineered host is *Pichia pastoris.*

9. A method for constructing the recombinant engineered host according to any of claims 6 to 8, comprising the following steps: transferring a coding gene of the recombinant fusion protein into a host through gene vector expression, virus vector expression, or a transgenic method; wherein the host is selected from the group consisting of the yeast, the CHO cell, the bacterium, the plant cell, the insect cell, and the animal cell.

10. A method for producing the recombinant fusion protein according to any of claims 1 to 5, comprising the following steps: a), constructing pYZ-hCBD/SA and rHSA/CBD recombinant plasmids with nucleotide sequences shown in SEQ ID NO: 2 and SEQ ID NO: 4, respectively; b), transforming the pYZ-hCBD/SA and rHSA/CBD recombinant plasmids separately into *Pichia pastoris* X33 competent cells; c), screening a recombinant yeast engineered strain expressing a specific protein; and d), conducting large-scale and high-density expression on a recombinant fusion protein that produces two molecular structures.

11. A pharmaceutical composition based on the recombinant fusion protein according to any of claims 1 to 5, wherein the pharmaceutical composition is coupled with a chemotherapy drug or a tumor cytotoxic protein using the recombinant fusion protein as a matrix.

12. The pharmaceutical composition according to claim 11, wherein the chemotherapy drug is selected from the group consisting of a platinum drug, a nitrosourea drug, an antifolate drug, an antipyrimidine drug, a vinblastine drug, a taxane drug, a camptothecin drug, an anthracycline drug, a drug that disrupts structure and function of DNA, a drug that is intercalated into DNA and interferes with transcription of the DNA, and a drug that affects protein synthesis.

13. The pharmaceutical composition according to claim 11, wherein the tumor cytotoxic protein is selected from the group consisting of ricin, diphtheria toxin, trichosanthin apoptin, Pseudomonas aeruginosa exotoxin, an inhibitor of apoptosis protein, human epidermal growth factor, gonadotropin-releasing hormone, and deoxyribonuclease II.

14. Use of the pharmaceutical composition according to any one of claims 11 to 13 in preparation of a drug for specific targeted treatment of a solid tumor.

15. A drug for specific targeted treatment of a solid tumor, wherein an active ingredient of the drug is the pharmaceutical composition according to claims 11 to 13.

16. A method for specific targeted treatment of a solid tumor, comprising: administering the drug according to claim 15 to a patient with the solid tumor.
